# EUROPÄISCHE PATENTSCHRIFT

(11) **EP 2 259 754 B1**
(45) Veröffentlichungstag und Bekanntmachung des Hinweises auf die Patenterteilung: **12.08.2015**
(21) Anmeldenummer: 09728573.8
(22) Anmeldetag: 12.02.2009
(51) Int. Cl.: A61F 2/38

(54) **KNIEENDOPROTHESE**
KNEE ENDOPROSTHESIS
ENDOPROTHÈSE DU GENOU

(30) Priorität: 05.04.2008 DE 102008017394
(43) Veröffentlichungstag der Anmeldung: 15.12.2010
(73) Patentinhaber: Aesculap AG, 78532 Tuttlingen (DE)
(72) Erfinder: HAGEN, Thomas, 78532 Tuttlingen (DE)
(74) Vertreter: Hoeger, Stellrecht & Partner Patentanwälte mbB
(86) Internationale Anmeldenummer: PCT/EP2009/000976
(87) Internationale Veröffentlichungsnummer: WO 2009/121450

(56) Entgegenhaltungen:
- DE-A1- 3 908 958
- DE-A1- 10 012 060
- US-A1- 2006 195 195
- US-A1- 2008 033 567

## Beschreibung

Die Erfindung betrifft eine Knieendoprothese mit einem Tibiateil, mit einem zwei Kondylenflächen aufweisenden Femurteil und mit einem zwischen Femurteil und Tibiateil angeordneten Meniskusteil, welches an seiner Oberseite zwei Lagerschalen zur Aufnahme und Lagerung der Kondylenflächen des Femurteiles und welches an seiner Unterseite eine Meniskus-Lagerfläche aufweist, die verschiebbar auf einer Tibia-Lagerfläche an der Oberseite des Tibiateiles aufliegt.

Eine Knieendoprothese dieser Art ist beispielsweise in der US 6,013,103 beschrieben. Bei dieser vorbekannten Knieendoprothese ist die Tibia-Lagerfläche eben ausgebildet und ruht auf der ebenen Oberseite des Tibiateils auf, die Verschiebung des Meniskusteils gegenüber dem Tibiateil erfolgt dabei als reine Parallelverschiebung des Meniskusteils parallel zur Ebene der Tibia-Lagerfläche und der Oberseite des Tibiateils. Auch bei unikondylären Knieendoprothesen ist es bekannt, ein Meniskusteil, das in diesem Falle nur eine Lagerschale für die Lagerung einer Femurkondyle aufweist, mit einer ebenen Meniskus-Lagerfläche auf einer ebenfalls ebenen Tibia-Lagerfläche zu lagern (EP 1 584 309 A1).

Bei Knieendoprothesen dieser Art kann das Meniskusteil gegenüber dem Tibiateil nach verschiedenen Richtungen verschoben und auch gedreht werden, wobei die Drehung in der Regel um die Mitte des Meniskusteiles erfolgt. Dazu sind zum Teil zapfenförmige Führungen vorgesehen (US 6,013,103). Es ist aber auch bekannt, das Meniskusteil auf dem Tibiateil so zu lagern, dass eine Drehung um eine Drehachse erfolgt, die gegenüber der Mitte des Meniskusteiles in medialer Richtung versetzt ist (US 6,013,103). Um dies zu erreichen, müssen für das Meniskusteil am Tibiateil spezielle Führungen vorgesehen sein, beispielsweise seitliche Wände.

In der US2006/0195195 A1 ist eine Knieendoprothese offenbart mit gekrümmten Stützflächen zwischen dem Meniskusteil und dem Tibiateil, wobei sie mit einem Zapfen um eine Achse rotierbar verbunden sind und die Achse nicht mittig angebracht ist.

In der US2008/0033567 A1 ist eine Knieendoprothese offenbart, in der das Meniskusteil mit einem Kugellager auf dem Tibiateil befestigt ist.

In der Praxis hat sich herausgestellt, dass mit Knieendoprothesen dieser Bauart zwar die anatomischen Verhältnisse relativ gut nachgebildet werden können, es können sich aber Schwierigkeiten dadurch ergeben, dass diese spezielle Konstruktion den erreichbaren Beugungswinkel zwischen Femur und Tibia begrenzt, da das Meniskusteil, das sich gegenüber dem Tibiateil nur in einer Ebene bewegt, die Bewegung der Knochen begrenzt, bei starken Beugungswinkeln können die Knochen am Meniskusteil anschlagen, so dass dadurch der Beugewinkel begrenzt wird.

Es ist Aufgabe der Erfindung, eine gattungsgemäße Knieendoprothese so auszubilden, dass sie bei einer optimalen Nachbildung der anatomischen Eigenschaften des gesunden Kniegelenkes insbesondere auch einen hohen Beugungswinkel zulässt.

Diese Aufgabe wird bei einer Knieendoprothese der eingangs beschriebenen Art erfindungsgemäß dadurch gelöst, dass die Meniskus-Lagerfläche und die Tibia-Lagerfläche gegenüber ihrer Mitte in medialer oder lateraler Richtung versetzt jeweils einen balligen Vorsprung oder einen balligen Rücksprung aufweisen, dass der ballige Vorsprung an einer der beiden Lagerflächen in den balligen Rücksprung der anderen Lagerfläche eintaucht und dadurch eine kugellagerartige Lagerung des Meniskusteils am Tibiateil bildet, dass im lateralen beziehungsweise medialen Teil der beiden Lagerflächen diese Lagerflächen aneinander anliegende Stützbereiche ausbilden, die in dorsal-ventraler Richtung gekrümmt sind und dort einen Krümmungsradius aufweisen, der mindestens um den Faktor 2,5 größer ist als der Krümmungsradius der balligen Vor- und Rücksprünge.

Bei einer solchen Ausgestaltung bilden die kugellagerartig ineinander greifenden Vor- und Rücksprünge des Meniskusteils und des Tibiateils eine kugellagerartige Lagerung, durch die bei einer Drehung des Meniskusteils auf dem Tibiateil eine Drehachse definiert wird, die in medialer oder lateraler Richtung gegenüber der Mitte des Meniskusteils versetzt ist, außerdem erlaubt diese kugellagerartige Lagerung auch zusätzlich zu der Verdrehung des Meniskusteils relativ zum Tibiateil eine Verschwenkung des Meniskusteils auf dem Tibiateil, die Schwenkachse verläuft dabei in medial-lateraler Richtung und damit im Wesentlichen senkrecht zu der durch die kugellagerartige Lagerung gebildeten Drehachse, die ihrerseits im Wesentlichen parallel zur Tibia-Längsachse angeordnet ist. Durch diese Verschwenkung kann das Meniskusteil sowohl in ventraler als auch in dorsaler Richtung an seinen Außenkanten abgesenkt werden, und dadurch wird die Gefahr herabgesetzt, dass das Meniskusteil die Beugung des Femurs gegenüber der Tibia auch bei großen Beugungswinkeln behindert, d.h. es werden größere Beugungswinkel möglich. Gleichzeitig wird erreicht, dass das Meniskusteil bei einer Drehung um eine Achse, die im Wesentlichen parallel zur Tibia-Längsachse verläuft, um einen Drehpunkt gedreht wird, der gegenüber der Mitte des Meniskusteils in medialer oder lateraler Richtung versetzt ist und somit den anatomischen Gegebenheiten des gesunden Kniegelenkes oder den Anforderungen des individuellen Bandapparates weitgehend entspricht.

Durch die beschriebenen Konstruktionsmerkmale besteht also für das Meniskusteil eine mehrdimensionale Bewegungsmöglichkeit, die über die reine Parallelverschiebung in einer Ebene hinausgeht und die außerdem in relativ einfacher Weise eine definierte Drehung um einen außerhalb der Mitte liegenden Drehpunkt sicherstellt. Die kugellagerartige Lagerung von Meniskusteil und Tibiateil ermöglicht nicht nur die Drehung um diese parallel zur Tibia-Längsachse verlaufende Drehachse, sondern auch die Schwenkung um die quer dazu verlaufende in medial-lateraler Richtung verlaufende Schwenkachse, wobei in jedem Falle die kugellagerartige Lagerung beibehalten wird.

Die Verdrehung und Verschwenkung des Meniskusteils gegenüber dem Tibia-teil erfolgt im Übrigen dadurch, dass bei der Verschwenkung des Femurs die Kondylenflächen des Femurs in den Lagerschalen an der Oberseite des Menis-kusteiles abgewälzt und verschoben werden, die Kondylenflächen üben dabei seitliche Kräfte auf das Meniskusteil aus, welche das Meniskusteil gegenüber dem Tibiateil verschieben und dabei zwangsläufig auch verdrehen und verschwenken. Insbesondere erhält man auf diese Weise auch bei der Beugung des Femurs gegenüber der Tibia eine Verdrehung der Tibia um die Längsachse, die sich dadurch ergibt, dass das Meniskusteil um die kugellagerartige Lagerung verdreht wird. Auch dies entspricht den anatomischen Gegebenheiten des natürlichen Kniegelenkes.

Durch den größeren Krümmungsradius der Stützbereiche gegenüber dem Krümmungsradius der balligen Vorsprünge und Rücksprünge wird sichergestellt, dass das Meniskusteil und das Tibiateil im Bereich der Vorsprünge und Rücksprünge ihre kugellagerartige Lagerung beibehalten, wenn das Meniskusteil gegenüber dem Tibiateil verschoben wird, so dass diese Verschiebung in eine Verdrehung und Verschwenkung des Meniskusteils gegenüber dem Tibiateil überführt wird, die Lagerung im Bereich der Vor- und Rücksprünge bleibt dabei erhalten.

Es kann vorgesehen sein dass die Stützbereiche nur in dorsal-ventraler Richtung gekrümmt sind, quer dazu jedoch nicht. Dies führt allerdings dazu, dass beim Verdrehen des Meniskusteils gegenüber dem Tibiateil eine vollflächige Anlage der Stützbereiche nicht über den gesamten Drehbereich sichergestellt sein kann, eine derartige vollflächige Anlage kann nur in bestimmten Winkelbereichen erreicht werden.

Eine bessere Übereinstimmung der Anlageflächen der Stützbereiche lässt sich erreichen, wenn gemäß einer bevorzugten Ausführungsform vorgesehen ist, dass die Stützbereiche der beiden Lagerflächen auch in lateral-medialer Richtung gekrümmt sind. Außerdem hat eine derartige Ausgestaltung noch eine stabilisierende Wirkung, da durch die zusätzliche Krümmung der Stützbereiche in lateral-medialer Richtung der seitlichen Verschiebung des Meniskusteils gegenüber dem Tibiateil in lateral-medialer Richtung entgegengewirkt wird.

Bei einer ersten bevorzugten Ausführungsform ist dabei vorgesehen, dass die Stützbereiche im lateralen Teil der beiden Lagerflächen Teile einer Kugeloberfläche sind, deren Radius mindestens um den Faktor 2,5 größer ist als der Radius der balligen Vor- und Rücksprünge, wobei die Mittelpunkte der balligen Vorsprünge gegenüber der Mitte der Lagerflächen in medialer oder lateraler Richtung und der Mittelpunkt der Kugeloberfläche dann in lateraler beziehungsweise medialer Richtung versetzt sind. Bei der Ausbildung der Stützbereiche als Kugeloberfläche lässt sich eine weitgehend flächige Anlage der Stützbereiche über einen größeren Winkelbereich annähern, allerdings ist auch in diesem Falle nicht über den gesamten Drehbereich des Meniskusteils eine derartige flächige Anlage in großen Bereichen zu gewährleisten.

Es ist daher günstig, wenn gemäß einer weiteren bevorzugten Ausführungsform die Stützbereiche längs einer oder mehrerer Linien verlaufen, die sich als Schnittlinie von zwei Zylinderflächen ergeben, nämlich einer senkrechten Zylinderfläche, deren Mittelachse durch den Mittelpunkt der balligen Vor- und Rücksprünge hindurchgeht und im Wesentlichen parallel zur Tibialängsachse verläuft, und einer horizontalen Zylinderfläche, deren Mittelachse durch den durch die in dorsal-ventraler Richtung verlaufende Krümmung definierten Mittelpunkt hindurchgeht und im Wesentlichen in medial-lateraler Richtung verläuft. Wenn die Stützbereiche in dieser Weise ausgebildet sind, ergibt sich auch bei Drehung des Implantatteils gegenüber dem Tibiateil um eine Drehachse, die mit der Mittelachse der senkrechten Zylinderfläche übereinstimmt, über einen größeren Winkelbereich eine vollflächige Anlage der Stützbereiche, so dass dadurch auch die Flächenpressung und damit der Abrieb reduziert werden können.

Bei einer besonders bevorzugten Ausführungsform ist vorgesehen, dass die Vor- und Rücksprünge und die Stützbereiche im Wesentlichen bis zu einer gemeinsamen Horizontalebene des Meniskusteils reichen, also etwa in derselben Höhe des Meniskusteils enden.

Die kugellagerförmige Lagerung des Meniskusteils am Tibiateil, die durch Rücksprünge und in diese eingreifende Vorsprünge erreicht wird, kann sowohl einen Vorsprung am Meniskusteil und einen entsprechenden Rücksprung am Tibiateil als auch einen Vorsprung am Tibiateil und einen entsprechenden Rücksprung am Meniskusteil umfassen, hier ist also eine Umkehr möglich. Dasselbe gilt hinsichtlich der Krümmung der Stützbereiche in lateral-medialer Richtung, diese Krümmung kann so ausgebildet sein, dass der Stützbereich am Tibiateil nach oben oder nach unten gewölbt ist, während dann natürlich die Wölbung in lateral-medialer Richtung bei dem Meniskusteil entgegengesetzt verläuft.

Die Krümmung der Stützbereiche in ventral-dorsaler Richtung hingegen muss immer so gewählt sein, dass das Meniskusteil beim Verschwenken in ventraler Richtung die ventrale Außenkante absenkt und beim Verschwenken in dorsaler Richtung die dorsale Außenkante, d.h. diese Krümmung muss immer so verlaufen, dass der Krümmungsmittelpunkt unterhalb der Lagerfläche zwischen Meniskusteil und Tibiateil angeordnet ist.

Die nachfolgende Beschreibung bevorzugter Ausführungsformen der Erfindung dient im Zusammenhang mit der Zeichnung der näheren Erläuterung. Es zeigen:
- Figur 1:: eine perspektivische schematische Ansicht eines Tibiateils und eines im Abstand dazu angeordneten Meniskusteils in einer Blickrichtung auf die Oberseite des Tibiateils;

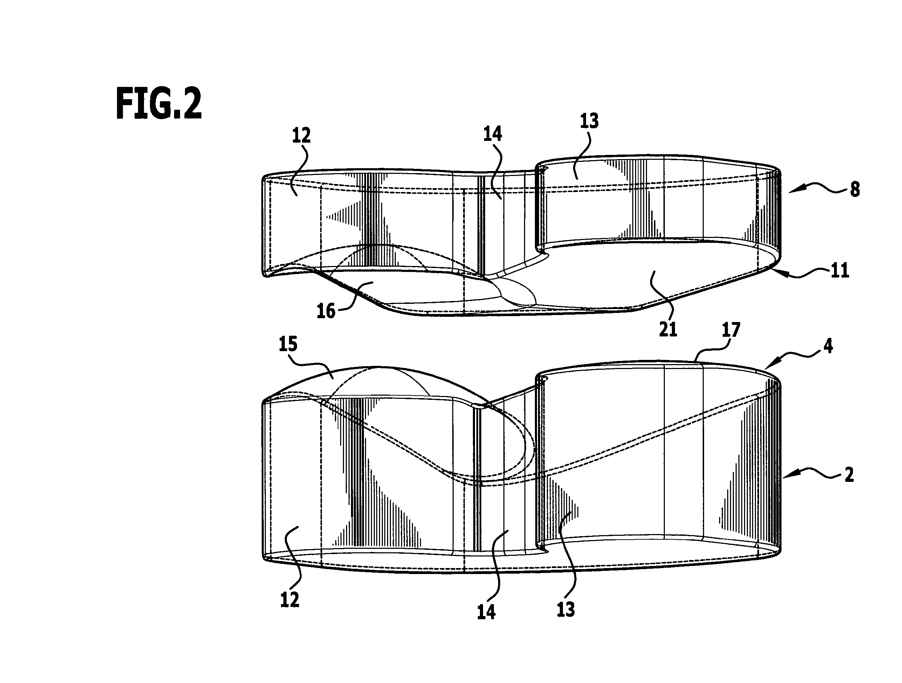

- Figur 2:: eine Ansicht ähnlich Figur 1 mit einer Blickrichtung gegen die Unterseite des Meniskusteils;

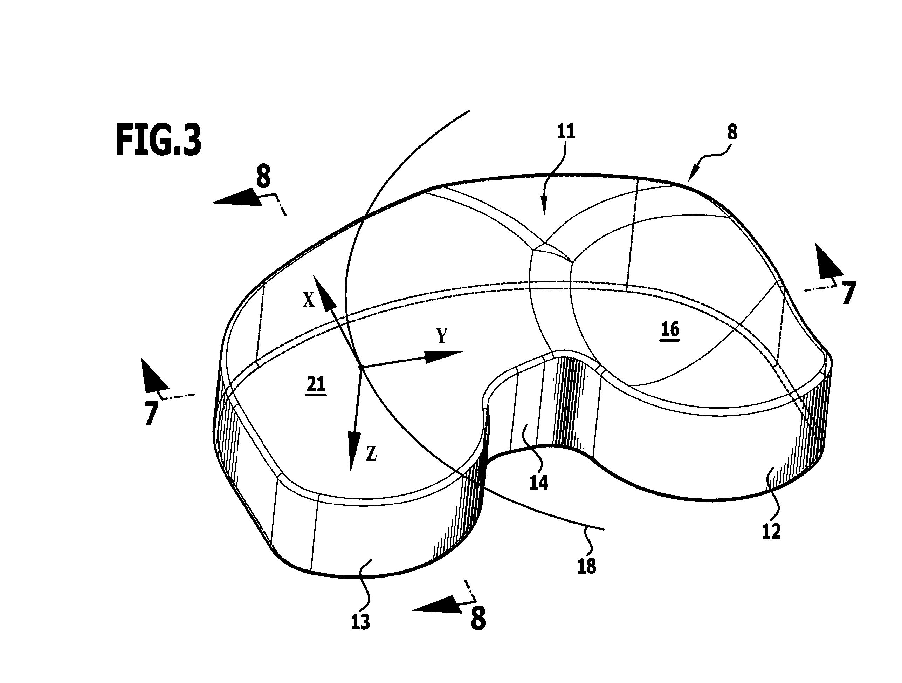

- Figur 3:: eine perspektivische Ansicht der Unterseite des Meniskusteils;

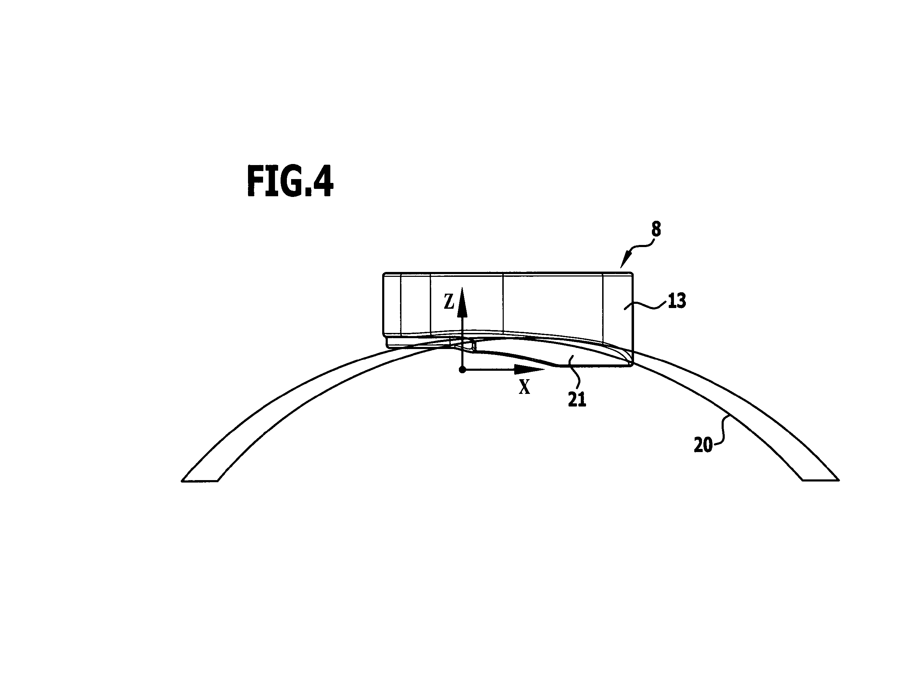

- Figur 4:: eine Seitenansicht des Meniskusteils mit einer Zylinderfläche zur Darstellung der Krümmung des Stützbereiches des Meniskusteils in dorsal-ventraler Richtung;

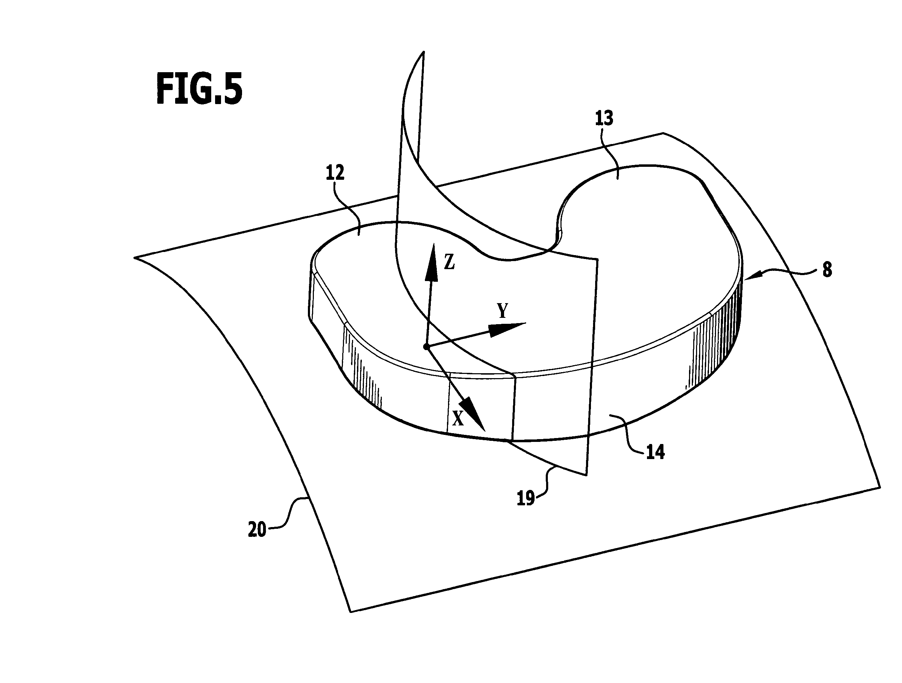

- Figur 5:: eine perspektivische Draufsicht auf das Meniskusteil mit der Zylinderfläche der Figur 4 und zusätzlich einer Zylinderfläche eines senkrechten Zylinders, der koaxial zur Drehachse des Meniskusteils gegenüber dem Tibiateil angeordnet ist;

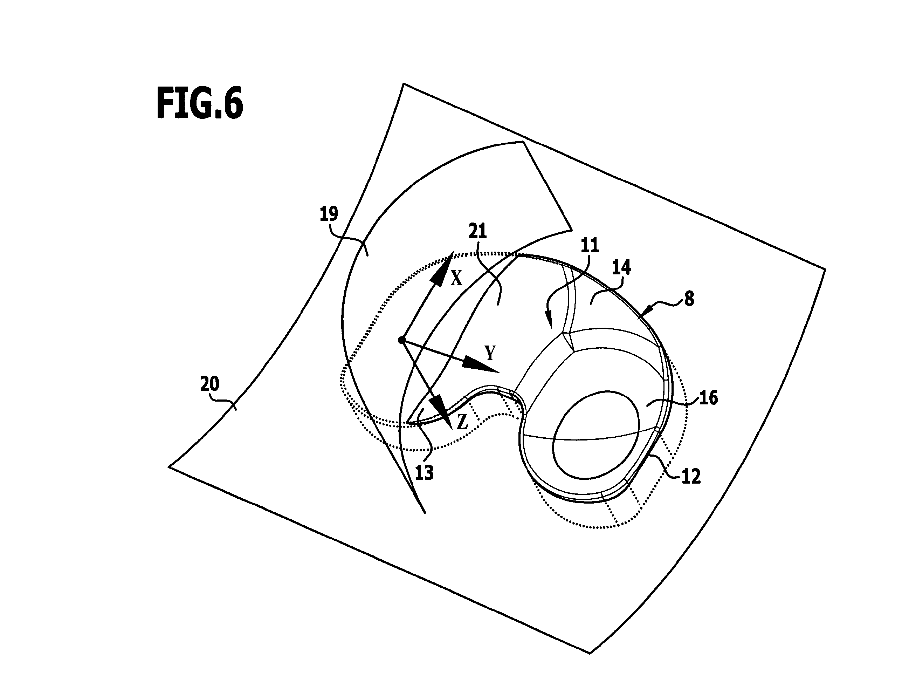

- Figur 6:: eine perspektivische Ansicht des Meniskusteils der Figur 5 von dessen Unterseite her mit Darstellung der beiden Zylinderflächen der Figur 5;

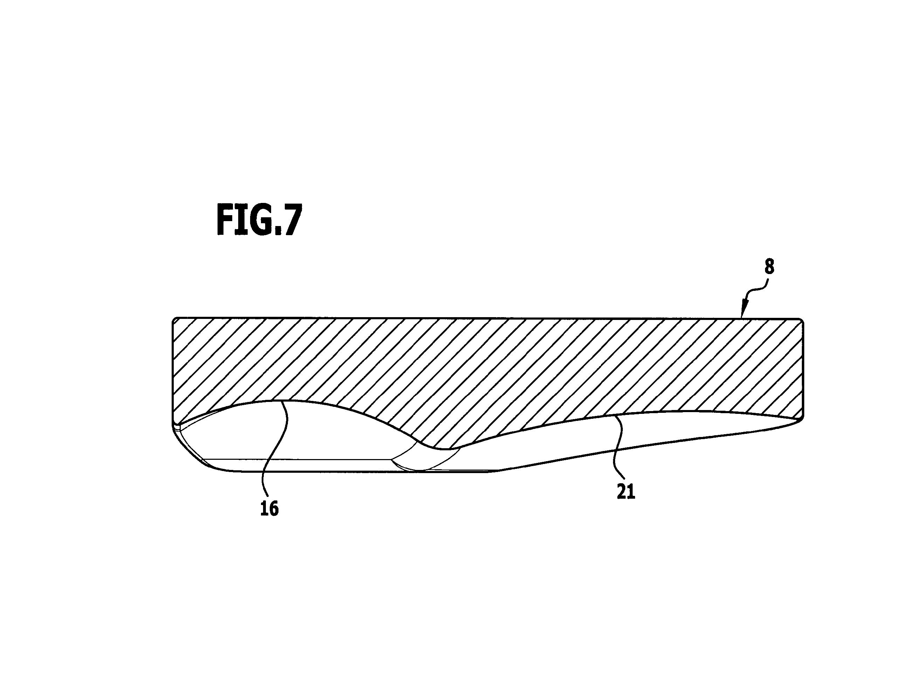

- Figur 7:: eine Schnittansicht des Meniskusteils der Figur 3 längs Linie 7-7 in Figur 3 und

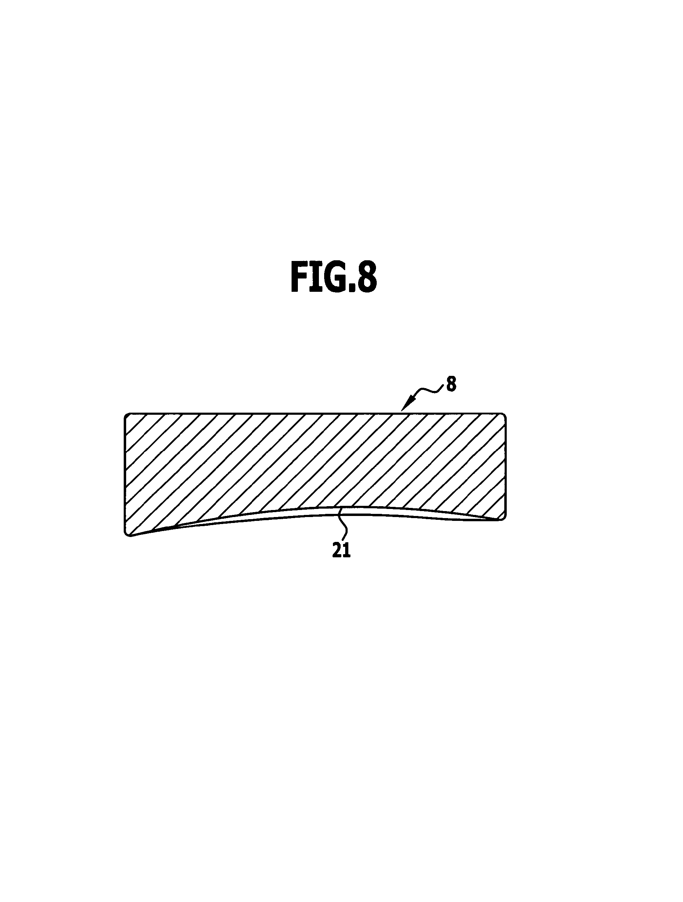

- Figur 8:: eine Schnittansicht längs Linie 8-8 in Figur 3.

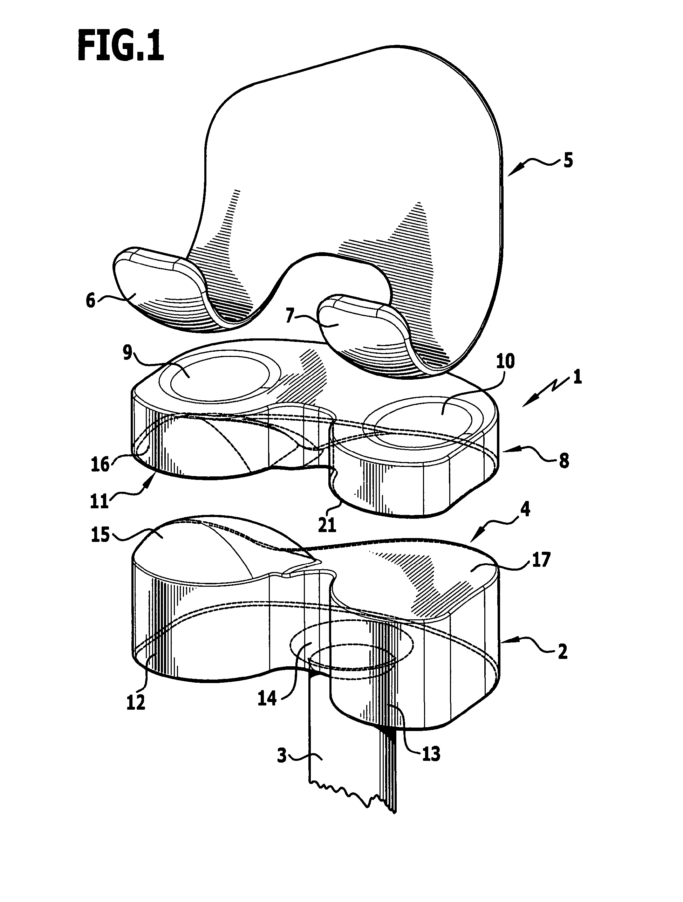

Die in Figur 1 dargestellte Knieendoprothese 1 umfasst ein Tibiateil 2 mit einem Stiel 3 zur Festlegung des Tibiateils 2 an einer Tibia und mit einer als Lagerfläche 4 dienenden Oberseite, weiterhin ein Femurteil 5 zur Festlegung am distalen Ende eines Femurs mit zwei nebeneinander liegenden, gewölbten Kondylenflächen 6, 7 und ein zwischen dem Tibiateil 2 und dem Femurteil 5 angeordnetes Meniskusteil 8, welches an seiner Oberseite zwei nebeneinander angeordnete Lagerschalen 9, 10 aufweist, in welche die Kondylenflächen 6, 7 eintauchen, und mit einer Meniskus-Lagerfläche 11 an der Unterseite des Meniskusteils 8.

Die Kondylenflächen 6, 7 können in den Lagerschalen 9, 10 so verschoben werden, dass einerseits ein Verschwenken des Femurs gegenüber der Tibia möglich ist und zum anderen auch in gewissem Umfange eine seitliche Verschiebung der Kondylenflächen 6, 7 in Längsrichtung der Lagerschalen 9, 10, dies ermöglicht eine kombinierte Roll-Gleit-Bewegung zwischen Meniskusteil 8 einerseits und Femurteil 5 andererseits. Da die Ausbildung der Lagerschalen 9, 10 und der Kondylenflächen 6, 7 für die vorliegende Erfindung nicht von vorrangiger Bedeutung ist, sind die Lagerschalen 9, 10 und das Femurteil 5 mit den Kondylenflächen 6, 7 nur in Figur 1 dargestellt, in den nachfolgenden Figuren beschränkt sich die Darstellung auf das Meniskusteil 8 und das Tibiateil 2 (ohne Stiel), dabei sind die Lagerschalen 9, 10 auf der Oberseite des Meniskusteils 8 nicht dargestellt, sondern die Oberseite des Meniskusteils 8 ist eben dargestellt, tatsächlich ist jedoch von einer Ausgestaltung gemäß der Darstellung der Figur 1 mit Lagerschalen 9, 10 an der Oberseite auszugehen.

Sowohl das Tibiateil 2 als auch das Meniskusteil 8 sind im Wesentlichen U-förmig ausgebildet mit zwei nebeneinander liegenden Seitenteilen 12, 13 und einem diese verbindenden stegförmigen Zwischenteil 14. Die Tibia-Lagerfläche 4 weist in dem medialen Seitenteil 12 an ihrer Oberseite einen kugeligen Vorsprung 15 auf, der Mittelpunkt des kugeligen Vorsprunges 15 ist gegenüber der Mitte des Tibiateils 2, die zwischen den beiden Seitenteilen 12, 13 liegt, in medialer Richtung versetzt und befindet sich etwa in der Mitte des medialen Seitenteiles 12, der Radius des kugeligen Vorsprunges 15 liegt im Bereich zwischen 20 mm und 30 mm, vorzugsweise im Bereich zwischen 22 mm und 26 mm.

Im medialen Seitenteil 12 des Meniskusteils 8 befindet sich ein zum Vorsprung 15 komplementärer Rücksprung 16, in den der Vorsprung 15 eintaucht, wenn das Meniskusteil 8 unmittelbar auf dem Tibiateil 2 aufliegt. Dadurch wird eine kugellagerartige Lagerung zwischen dem Tibiateil 2 einerseits und dem Meniskusteil 8 andererseits ausgebildet. Durch diese Lagerung kann einmal das Meniskusteil 8 um eine Drehachse verdreht werden, die im Wesentlichen parallel zur Tibia-Längsachse verläuft und somit im Wesentlichen senkrecht zu der Oberseite des Meniskusteils 8 angeordnet ist. Es ist aber außerdem auch durch diese kugellagerförmige Lagerung möglich, das Meniskusteil gegenüber dem Tibiateil zu verschwenken, beispielsweise um eine Schwenkachse, die in medial-lateraler Richtung verläuft.

Im Bereich des lateralen Seitenteiles 13 ist die Oberseite des Tibiateiles 2 als Stützbereich 17 ausgebildet. In diesem Stützbereich hat die Tibia-Lagerfläche 4 eine gekrümmte Kontur, und zwar ist dieser Stützbereich 17 sowohl in ventral-dorsaler Richtung gekrümmt als auch in medial-lateraler Richtung. In beiden Fällen ist die Krümmung bei dem in der Zeichnung dargestellten Ausführungsbeispiel so gewählt, dass die Krümmungsmittelpunkte unterhalb des Stützbereiches 17 liegen, der Krümmungsradius ist dabei bei beiden Krümmungen relativ groß, bei dem dargestellten Ausführungsbeispiel ist der Krümmungsradius in ventral-dorsaler Richtung etwa um den Faktor 2,5 größer als der Krümmungsradius der kugelig geformten Vorsprünge 15 und Rücksprünge 16. In lateral-dorsaler Richtung ist der Krümmungsradius des Stützbereichs 17 etwa um den Faktor 2,5 bis 3 größer als der Krümmungsradius des Vorsprunges 15 und des Rücksprunges 16.

Die genaue Kontur des Stützbereiches 17 kann unterschiedlich gewählt werden, beispielsweise können die Krümmungen in ventral-dorsaler Richtung gleich sein wie die Krümmungen in medial-lateraler Richtung, so dass man im Wesentlichen einen kugeligen Stützbereich 17 erhält. Es ist aber auch möglich, dass der Stützbereich 17 die Form eines Torus aufweist, allerdings eines Torus, dessen Längsachse in Umfangsrichtung zusätzlich aus der Ebene heraus gekrümmt ist, wie dies der Krümmung in ventral-dorsaler Richtung entspricht.

Bei dem in der Zeichnung dargestellten Ausführungsbeispiel ist eine besondere Form des Stützbereiches 17 gewählt. Dieser Stützbereich 17 wird nämlich aufgespannt durch eine Vielzahl von Schnittlinien 18, die sich durch die Durchdringung von zwei Zylindern ergeben, nämlich einem senkrechten Zylinder 19 und einem horizontalen Zylinder 20.

Der senkrechte Zylinder 19 hat eine Längsmittellinie, die durch den höchsten Punkt des Vorsprunges 15 hindurchgeht und im Wesentlichen parallel zur Tibia-Längsachse verläuft, diese Achse bildet die Drehachse des Meniskusteiles 8 gegenüber dem Tibiateil 2, wenn diese Teile gegeneinander verdreht werden.

Der horizontale Zylinder 20 beschreibt die Krümmung in dorsal-ventraler Richtung, die Längsmittellinie des horizontalen Zylinders 20 verläuft in lateral-medialer Richtung und der Radius des horizontalen Zylinders 20 entspricht dem Krümmungsradius der Krümmung des Stützbereiches 17 in dorsal-ventraler Richtung. Zwischen dem horizontalen Zylinder 20 und dem senkrechten Zylinder 19 mit unterschiedlichem Radius ergeben sich eine Vielzahl von Schnittlinien 18, die dann gemeinsam den Stützbereich 17 aufbauen.

Die Meniskus-Lagerfläche 11 ist komplementär zur Tibia-Lagerfläche 4 ausgebildet, so dass sich bei der Anlage des Meniskusteiles 8 am Tibiateil 2 eine im Wesentlichen vollflächige Anlage einmal im Bereich des Vorsprunges 15 und des Rücksprunges 16 und zum anderen im Bereich des Stützbereiches 17 ergibt, in diesem Stützbereich 17 liegt das Meniskusteil 8 mit einem zum Stützbereich 17 komplementären Stützbereich 21 an diesem an.

Durch die beschriebene Lagerung ergibt sich beim Ausüben von horizontalen Kräften auf das Meniskusteil 8 die Möglichkeit für das Meniskusteil 8, sich gegenüber dem Tibiateil 2 zu bewegen, und zwar einmal in Form einer Drehung um die Drehachse, die im Wesentlichen mit der Mittelachse des senkrechten Zylinders 19 zusammenfällt, und zum anderen durch Verschwenken des Meniskusteils 8 um eine horizontale Schwenkachse, die im Wesentlichen senkrecht auf der Wand des senkrechten Zylinders 19 steht. In einer Mittelstellung verläuft diese Schwenkachse also im Wesentlichen in lateral-medialer Richtung, bei einer Verdrehung des Meniskusteils 8 gegenüber dem Tibiateil 2 wird diese Schwenkachse entsprechend dem Drehwinkel ebenfalls gedreht, und dies führt auch bei einer Drehung zu einer im Wesentlichen vollflächigen Anlage des Meniskusteils am Tibiateil. Gleichzeitig wird bei dieser Drehung das Meniskusteil um diese Schwenkachse verschwenkt, so dass die jeweils außen liegende Kante abgesenkt wird, und dadurch wird dem Gelenk ein Spielraum für einen größeren Beugungswinkel gegeben.

Die Drehung um die Drehachse erfolgt dabei also immer um eine Drehachse, die außermittig angeordnet ist und in medialer Richtung versetzt ist, die Verschwenkung erfolgt zusätzlich zwangsgeführt um eine Schwenkachse, die im Wesentlichen horizontal verläuft und ihren Winkel gegenüber der mediallateralen Richtung mit dem Drehwinkel des Meniskusteils ändert.

Die Kräfte, die zu einer Verschiebung des Meniskusteils gegenüber dem Tibiateil führen, werden im Wesentlichen durch das Femurteil auf das Meniskusteil übertragen, wenn das Gelenk gebeugt wird, insbesondere übertragen also die Kondylenflächen 6, 7 über die Lagerschalen 9, 10 derartige Kräfte, die zu einer Drehung und Verschwenkung des Meniskusteils gegenüber dem Tibiateil führen.

Das Tibiateil 2 und das Femurteil 5 bestehen in an sich bekannter Weise normalerweise aus einem körperverträglichen Metall, beispielsweise aus Titan oder einer Titanlegierung, während das Meniskusteil vorzugsweise aus einem sterilisierbaren und körperverträglichen Kunststoff besteht, beispielsweise aus Hochdruckpolyethylen. Durch die beschriebene Formgebung der Lagerflächen wird eine vollflächige Anlage im Bereich zwischen dem Tibiateil und dem Meniskusteil erreicht, und dies führt zu der Vermeidung von Kraftspitzen und zur Reduzierung der Abnutzung der aneinander anliegenden Lagerflächen.

Bei dem in der Zeichnung dargestellten Ausführungsbeispiel sind der Vorsprung 15 und der Rücksprung 16 gegenüber der Mitte der Lagerfläche in medialer Richtung versetzt, der Stützbereich dagegen in lateraler Richtung. Es ist grundsätzlich auch möglich, dass eine umgekehrte Anordnung gewählt wird, dann sind der Vorsprung 15 und der Rücksprung 16 in lateraler Richtung versetzt und der Stützbereich 17 dagegen in medialer Richtung. Dadurch wird natürlich auch die Drehachse des Meniskusteils 8 entsprechend verlagert. Der häufigere Fall ist die Anordnung der Drehachse im medialen Teil der Lagerfläche, es kann aber - insbesondere aufgrund einer Änderung der Bandstruktur - notwendig sein, die Drehachse nicht in medialer Richtung sondern in lateraler Richtung gegenüber der Mitte der Lagerfläche zu versetzen.

## Patentansprüche

1. Knieendoprothese (1) mit einem Tibiateil (2), mit einem zwei Kondylenflächen (6, 7) aufweisenden Femurteil (5) und mit einem zwischen Femurteil (5) und Tibiateil (2) angeordneten Meniskusteil (8), welches an seiner Oberseite zwei Lagerschalen (9, 10) zur Aufnahme und Lagerung der Kondylenflächen (6, 7) des Femurteiles (5) und welches an seiner Unterseite eine Meniskus-Lagerfläche (11) aufweist, die verschiebbar auf einer Tibia-Lagerfläche (4) an der Oberseite des Tibiateils (2) aufliegt, wobei die Meniskus-Lagerfläche (11) und die Tibia-Lagerfläche (4) gegenüber ihrer Mitte in medialer oder lateraler Richtung versetzt jeweils einen Vorsprung (15) oder einen Rücksprung (16) aufweisen und der Vorsprung (15) an einer der beiden Lagerflächen (11, 4) in den Rücksprung (16) der anderen Lagerfläche (4, 11) eintaucht und dadurch eine Lagerung des Meniskusteils (8) am Tibiateil (2) bildet, wobei der Vorsprung (15) und der Rücksprung (16) ballig ausgebildet sind, so dass durch das Eintauchen des Vorsprunges (15) in den Rücksprung (16) eine kugellagerartige Lagerung des Meniskusteils (8) am Tibiateil (2) ausgebildet wird, und wobei im lateralen beziehungsweise medialen Teil (13) der beiden Lagerflächen (4, 11) diese aneinander anliegende Stützbereiche (17, 21) ausbilden, die in dorsalventraler Richtung gekrümmt sind und dort einen Krümmungsradius aufweisen, der mindestens um den Faktor 2,5 größer ist als der Krümmungsradius des balligen Vorsprunges (15) und des balligen Rücksprunges (16).

2. Knieendoprothese nach Anspruch 1, **dadurch gekennzeichnet, dass** die Stützbereiche (17, 21) der beiden Lagerflächen (4, 11) auch in lateralmedialer Richtung gekrümmt sind.

3. Knieendoprothese nach Anspruch 2, **dadurch gekennzeichnet, dass** die Stützbereiche (17, 21) der beiden Lagerflächen (4, 11) Teile einer Kugeloberfläche sind, deren Radius mindestens um den Faktor 2,5 bis 3 größer ist als der Radius des balligen Vorsprunges (15) und des balligen Rücksprunges (16), wobei die Mittelpunkte des balligen Vorsprunges (15) und des balligen Rücksprunges (16) gegenüber der Mitte der Lagerflächen (4, 11) in medialer Richtung und der Mittelpunkt der Kugeloberfläche in lateraler Richtung versetzt sind.

4. Knieendoprothese nach Anspruch 2, **dadurch gekennzeichnet, dass** die Stützbereiche (17, 21) längs einer oder mehrerer Linien verlaufen, die sich als Schnittlinie von zwei Zylinderflächen (19, 20) ergeben, nämlich einer senkrechten Zylinderfläche (19), deren Mittelachse durch den Mittelpunkt des balligen Vorsprunges (15) und des balligen Rücksprunges (16) hindurchgeht und im Wesentlichen parallel zur Tibialängsachse verläuft, und einer horizontalen Zylinderfläche (20), deren Mittelachse durch den durch die in dorsal-ventraler Richtung verlaufende Krümmung definierten Mittelpunkt hindurchgeht und im Wesentlichen in mediallateraler Richtung verläuft.

5. Knieendoprothese nach einem der voranstehenden Ansprüche, **dadurch gekennzeichnet, dass** der Vorsprung (15) und der Rücksprung (16) und die Stützbereiche (17, 21) im Wesentlichen bis zu einer gemeinsamen Horizontalebene des Meniskusteils (8) reichen.

## Claims

1. Knee endoprosthesis (1) comprising a tibia part (2), a femur part (5) having two condylar surfaces (6,7), and a meniscus part (8) arranged between the femur part (5) and the tibia part (2), the meniscus part having on its upper side two bearing shells (9, 10) for receiving and mounting the condylar surfaces (6, 7) of the femur part (5) and having on its underside a meniscus-bearing surface (11) which rests in a displaceable manner on a tibia-bearing surface (4) on the upper side of the tibia part (2), the meniscus-bearing surface (11) and the tibia-bearing surface (4) each having a projection (15) or a recess (16) offset in a medial or a lateral direction in relation to their center, and the projection (15) on one of the two bearing surfaces (11, 4) engaging the recess (16) of the other bearing surface (4, 11) and thereby forming a mounting of the meniscus part (8) on the tibia part (2), wherein the projection (15) and the recess (16) are formed spherically, so that by engagement of the projection (15) into the recess (16) a ball bearing-like mounting of the meniscus part (8) on the tibia part (2) is formed, and wherein in the lateral or medial part (13) of the two bearing surfaces (4, 11), these bearing surfaces form supporting areas (17, 21) bearing on each other, which are curved in a dorsal-ventral direction and have a radius of curvature there, which is greater than the radius of curvature of the spherical projection (15) and the spherical recess (16) by at least the factor 2.5.

2. Knee endoprosthesis in accordance with claim 1, **characterized in that** the supporting areas (17, 21) of the two bearing surfaces (4, 11) are also curved in a lateral-medial direction.

3. Knee endoprosthesis in accordance with claim 2, **characterized in that** the supporting areas (17, 21) of the two bearing surfaces (4, 11) are parts of a spherical surface whose radius is greater than the radius of the spherical projection (15) and the spherical recess (16) by at least the factor 2.5 to 3, the center points of the spherical projection (15) and the spherical recess (16) being offset in relation to the center of the bearing surfaces (4, 11) in a medial direction and the center point of the spherical surface being offset in a lateral direction.

4. Knee endoprosthesis in accordance with claim 2, **characterized in that** the supporting areas (17, 21) extend along one or more lines, which result as line of intersection of two cylinder surfaces (19, 20), namely a perpendicular cylinder surface (19) whose center axis passes through the center point of the spherical projection (15) and the spherical recess (16) and extends substantially parallel to the tibial longitudinal axis, and a horizontal cylinder surface (20) whose center axis passes through the center point defined by the curvature extending in a dorsal-ventral direction and extends substantially in a medial-lateral direction.

5. Knee endoprosthesis in accordance with any one of the preceding claims, **characterized in that** the projection (15) and the recess (16) and the supporting areas (17, 21) extend substantially as far as a common horizontal plane of the meniscus part (8).

## Revendications

1. Endoprothèse de genou (1) comprenant une partie tibiale (2), une partie de fémur (5) présentant deux surfaces condyliennes (6, 7), et une partie formant ménisque (8), qui est agencée entre la partie de fémur (5) et la partie tibiale (2), et comporte sur son côté supérieur, deux cavités de palier (9, 10) destinées à l'accueil et au montage articulé des surfaces condyliennes (6, 7) de la partie de fémur (5), et sur son côté inférieur, une surface de palier de ménisque (11) reposant de manière coulissante sur une surface de palier tibiale (4) du côté supérieur de la partie tibiale (2), endoprothèse
dans laquelle la surface de palier de ménisque (11) et la surface de palier tibiale (4) présentent, de manière décalée en direction médiale ou latérale par rapport à leur milieu, respectivement une proéminence (15) ou un retrait (16), et la proéminence (15) sur l'une des deux surfaces de palier (11, 4) s'engage dans le retrait (16) de l'autre surface de palier (4, 11) en réalisant ainsi un montage articulé de la partie formant ménisque (8) sur la partie tibiale (2),
dans laquelle la proéminence (15) et le retrait (16) sont de configuration bombée, de sorte que par l'engagement de la proéminence (15) dans le retrait (16), il est réalisé un montage articulé du type palier à rotule de la partie formant ménisque (8) sur la partie tibiale (2), et
dans laquelle dans la partie (13) latérale, respectivement médiale, des deux surfaces de palier (4, 11), celles-ci forment des zones d'appui (17, 21) appliquées mutuellement l'une contre l'autre, qui sont courbées dans la direction dorsale-ventrale et y présentent un rayon de courbure qui est plus grand, d'au moins un facteur 2,5, que le rayon de courbure de la proéminence (15) bombée et du retrait (16) bombé.

2. Endoprothèse de genou selon la revendication 1, **caractérisée en ce que** les zones d'appui (17, 21) des deux surfaces de palier (4, 11) sont également courbées dans la direction latérale-médiale.

3. Endoprothèse de genou selon la revendication 2, **caractérisée en ce que** les zones d'appui (17, 21) des deux surfaces de palier (4, 11) sont des secteurs d'une surface sphérique dont le rayon est plus grand, d'au moins un facteur 2,5 à 3, que le rayon de la proéminence (15) bombée et du retrait (16) bombé, les points centraux de la proéminence (15) bombée et du retrait (16) bombé étant décalés en direction médiale par rapport au milieu des surfaces de palier (4, 11), et le point central de la surface sphérique est décalé en direction latérale.

4. Endoprothèse de genou selon la revendication 2, **caractérisée en ce que** les zones d'appui (17, 21) s'étendent le long d'une ou de plusieurs lignes, qui résultent de la ligne d'intersection de deux surfaces de cylindre (19, 20), à savoir une surface de cylindre verticale (19), dont l'axe médian passe par le point central de la proéminence (15) bombée et du retrait (16) bombé et s'étend sensiblement de manière parallèle à l'axe longitudinal du tibia, et une surface de cylindre horizontale (20), dont l'axe médian passe par le point central de la courbure se déployant dans la direction dorsale-ventrale, et s'étend sensiblement dans la direction médiale-latérale.

5. Endoprothèse de genou selon l'une des revendications précédentes, **caractérisée en ce que** la proéminence (15) et le retrait (16) et les zones d'appui (17, 21) s'étendent sensiblement jusqu'à un plan horizontal commun de la partie formant ménisque (8).
